Europäisches Patentamt

European Patent Office

Office européen des brevets

(19)

(11) Publication number: **0 132 918**
A2

# (12) EUROPEAN PATENT APPLICATION

(21) Application number: **84303469.5**

(22) Date of filing: **22.05.84**

(51) Int. Cl.⁴: **G 11 B 15/66**
G 11 B 15/18, G 11 B 5/48
G 11 B 5/02

(30) Priority: 27.05.83 GB 8314687
27.05.83 GB 8314688
28.07.83 GB 8320431

(43) Date of publication of application:
13.02.85 Bulletin 85/7

(84) Designated Contracting States:
AT BE CH DE FR GB IT LI LU NL SE

(71) Applicant: SINCLAIR RESEARCH LIMITED
25 Willis Road
Cambridge CB1 2AQ(GB)

(72) Inventor: Southward, David Conner
The Old Thatch 60 Denmark Road
Cottenham Cambridge CB4 4QF(GB)

(72) Inventor: Cheese, Benjamin John
2, Auckland Cottages
Cambridge(GB)

(74) Representative: Abbott, David John et al,
Abel & Imray Northumberland House 303-306 High
Holborn
London, WC1V 7LH(GB)

(54) Magnetic tape recording and reproducing apparatus.

(57) A magnetic tape recording and reproducing apparatus uses a cassette having a pressure roller near one corner with an aperture in one adjacent edge for a drive roller to engage the tape and an aperture in the other adjacent edge for the tape to engage a transducing head. The drive roller and head are fixed in position and a spring presses the cassette towards the drive roller, the pressure on the pressure roller also serving to retain the cassette and locate it against fixed stops. The head has projections for aligning the tape and determining the position of the cassette. The head position is referenced to the casing by standing on a surface of the casing and being held in place by resilient arms secured to the casing. The head is connected to a read-write circuit using a push-pull amplifier powered by two current sources. Any one of a plurality of such apparatus can be selected using a chain of D-type flip-flops (one per apparatus) and applying a number of clock pulses corresponding the apparatus to be selected.

FIG.1.

Croydon Printing Company Ltd.

MAGNETIC TAPE RECORDING AND REPRODUCING APPARATUS

The present invention relates to magnetic tape recording and reproducing apparatus and especially to such apparatus which uses magnetic tape in a cassette or cartridge.

In conventional cassette magnetic tape recording and reproducing apparatus, it is customary for the cassette to be loaded into the apparatus and fixed in position therein and then for the head assembly and a pinch roller to be brought into contact with the tape so that the tape is driven by a drive roller across the head assembly. Such apparatus is relatively complicated and expensive to produce with close mechanical tolerances such as would be needed for high density recording on the tape.

It is an object of the present invention to provide improved magnetic tape recording and reproducing apparatus.

According to the present invention there is provided magnetic tape recording and reproducing apparatus using magnetic tape in a cassette and having a magnetic transducing head and tape driving means with a drive roller bearing against the tape and a pinch roller in the cassetts, wherein both the head and the drive roller are fixed in position in the apparatus, spring means is provided urging the cassette towards the drive roller and the drive roller is so disposed in relation to the cassette that the pressure of the drive roller serves to urge the cassette towards the head and also to retain the cassette in the apparatus.

The term "cassette" as used in this specification includes a magnetic tape cartridge.

The cassette itself includes a roller round which the tape runs which serves as a pinch roller so that the pressure of the drive roller is effective on the cassette.

The apparatus may include a first stop member beside the head and a second stop member beside the drive roller, which members under the influence of the spring means determine the position of the cassette when pushed home in the apparatus.

Because the cassette is urged by the spring means towards the drive roller, the cassette when it is inserted will tend to drive the roller round in a direction opposite to its direction of rotation when driving tape and by virtue of inertia of the rotational part of the drive means, for example an electric motor, the drive roller may still be rotating when it engages the tape when the cassette is pushed fully home in the apparatus. This could lead to the drive roller unwinding a loop of tape from the cassette which could result in the tape becoming creased or otherwise damaged with consequent degradation of the magnetic recording properties of the tape. This problem can be overcome in a number of ways, for example the reverse rotation of the drive reel may be prevented by a ratchet or lock which is released when the cassette is pushed fully home. Alternatively, some means may be provided to prevent the cassette contacting the drive roller until it is pressed fully home, which means may consist of a free running wheel, concentric with the drive roller which engages the casing of the cassette until it is pressed fully home. The drive means itself may incorporate a ratchet mechanism which prevents it being rotated in the reverse direction.

An intended use of apparatus according to the present invention is the recording and reproducing of digital data used in relation with a computer, and it is envisaged that the density of data recorded on the magnetic tape will be very high with the consequent result that it is essential for the gap of the magnetic transducing head to be accurately at right angles to the longitudinal direction of the tape. This accuracy is needed because the cassette is likely to have been recorded on a different machine from that on which it is replayed. To this end, the magnetic transducing head is provided with tape guide members which contact one or both edges of the tape both upstream and downstream of the head gap, thereby determining the alignment of the tape over the head itself. The tape guide members may also be used to determine the position of the cassette casing relative to the head so that the position of the tape does not have to be changed very much by the tape guide members.

The magnetic transducing head is preferably mounted in the apparatus in such a way that the longitudinal direction of the tape is parallel to a surface of the body of the apparatus, so that the alignment of the tape in the cassette with the desired direction over the head is more easily achieved. In order to position the head in the apparatus, it is proposed that it be provided with side plates with legs which are rested on the aforesaid surface of the body of the apparatus and that these legs are maintained in contact with this surface by means of resilient members, e.g. metallic strips, extending on either side of the head by which the head is secured to the surface using, for example, screws, rivets or plastics pegs.

The present invention also provides an electrical circuit which is particularly suited to the electrical conditions imposed by digital data processing apparatus and which is suitable for use in magnetic tape recording and reproducing apparatus.

As part of the present invention, an electrical circuit includes first and second input ports connected to controllable means, an output port connected to the controllable means, and first and second current sources connected to the controllable means, the electrical circuit being arranged to provide, from the controllable means, output values dependent on input signal values and at least one of the current sources.

In a first form of the electrical circuit, the first and second input ports are connected to control ports of respective first and second switch means, the first and second current sources are connected respectively to the first and second switch means which are so arranged that switching on the first switch means activates the first current source, to provide at an output port, the current from the first current source in a first sense and the current from the second current source in the opposite to the first sense.

In the above mentioned first form of the electrical circuit, the first and second switch means may be first and second bipolar transistors arranged with their base electrodes connected respectively to the first and second input ports, their emitter electrodes connected respectively to the first and second current sources, their collector electrodes connectible to one terminal of a voltage source the other terminal of which is connectible to the terminals of the current sources remote from the transistors, and the output port provided by the emitter electrodes of the transistors.

In a second form of the electrical circuit, the first and second input ports are connected to control ports of respective first and second amplifier means, the first and second current sources are connected to the respective first and second control ports of the amplifier means, to provide, at an output port, an imbalance output current dependent on a signal at an input port and the current sources.

In the above mentioned second form of the electrical circuit, the first and second amplifier means may be first and second bipolar transistors arranged with their base electrodes connected to a fixed voltage level, their emitter electrodes connected respectively to the first and second current sources, their collector electrodes connected to respective resistors which are connectible to one terminal of a voltage source the other terminal of which is connectible to the terminals of the current sources remote from the transistors, the input ports being provided by the collector electrodes of the transistors.

In a third form of the electrical circuit, the first and second input ports are connected, via respective resistors, to respective first and second control ports of amplifier means, the first and second current sources are connected respectively to the first and second control ports of the amplifier means, and an output value of the amplifier means is arranged to control the activation of one of the current sources, to provide, at an output port, an output value dependent on the current source controlled by the output value.

The first form of the electrical circuit, referred to above, employing bipolar transistors, may be changed to the second form of the electrical circuit, referred to above, by connecting the transistor base electrodes to a fixed voltage level. The bipolar transistor implementations of the first and second forms of the electrical circuit, in combination with switch means for effecting the change from one form to the other, and a magnetic head connected between the emitter electrodes of the transistors, act as data recording and data playback means, respectively, and, in practice, facilitate direct connection of the magnetic head to the transistors, thereby providing an extremely compact recording and playback arrangement.

Another aspect of the operation of data processing apparatus is the control of a plurality of peripheral devices, for example a plurality of data recording and reproducing units, by means of a common control unit.

In accordance with the above mentioned other aspect of the operation of data processing apparatus, a method of controlling a plurality of data recording and reproducing units by means of a common control unit includes the provision of an element of a serial data store in each of the data recording and reproducing units, the connection of the elements to form a seial data store with means for loading and clocking from the common control unit, and the provision of signals at the common control unit to load each element in turn, and arranging that each element when loaded activates the recording and reproducing unit containing it.

The method of controlling a plurality of recording and reproducing units leads to the provision of recording and reproducing apparatus including an element of a serial data store in control means for the apparatus.

In order that the invention may be fully understood and readily carried into effect it will now be described with reference to the accompanying drawings, of which:-

FIGURE 1 is a plan view of part of an apparatus according to an example of the present invention with a cassette inserted;

FIGURE 2 is a side view of the magnetic transducing head used in the apparatus shown in Figure 1;

FIGURE 3 is a plan view of the head shown in Figure 2;

FIGURE 4 is a circuit diagram representation of an electrical circuit which may be used in a first mode as a recording amplifier and in a second mode as a playback amplifier;

FIGURE 5 showing unequal current sources included in the electrical circuit;

FIGURE 6 is a diagrammatic representation of the magnetisation pattern which may result in a magnetic tape subjected to magnetisation by the circuit of Figure 2;

FIGURES 7 and 8 are diagrammatic representations of, respectively, data recorded on a magnetic tape after a.c. erasure and data recorded on a magnetic tape after d.c. erasure;

FIGURE 9 is a diagrammatic representation of a data recovery circuit employing hysteresis to detect gaps between data blocks in a magnetic tape;

FIGURE 10 is a diagrammatic representation of an electrical circuit for providing hysteresis in the data recovery circuit of Figure 9;

FIGURES 11 and 12 are diagrammatic representations of the pattern of recorded data on a magnetic tape and the recovered data provided by the data recovery circuit of Figure 9;

FIGURE 13 is a block diagram representation of an arrangement for controlling a plurality of recording and playback units from a common control unit in data processing apparatus; and

FIGURE 14 is a schematic representation of the control signals from the common control unit to the units of Figure 13.

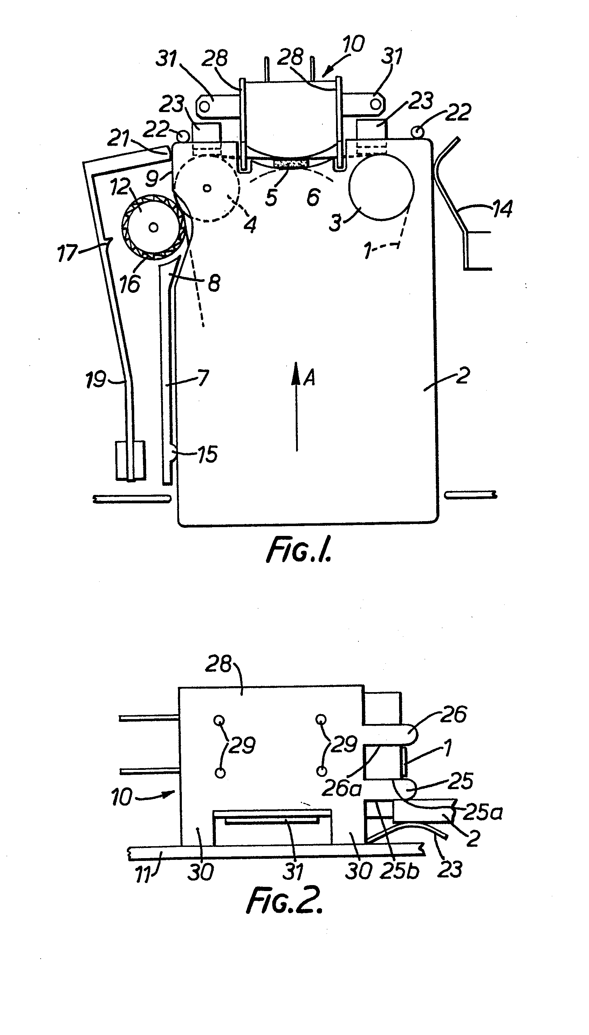

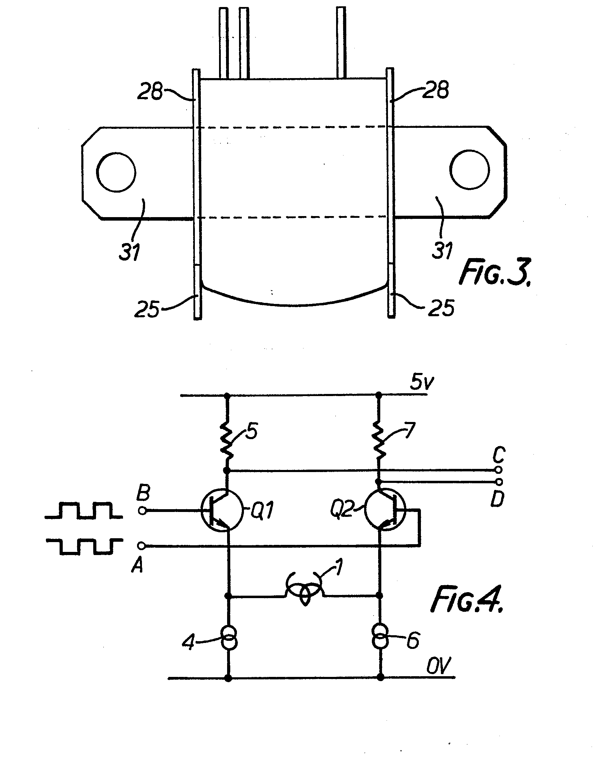

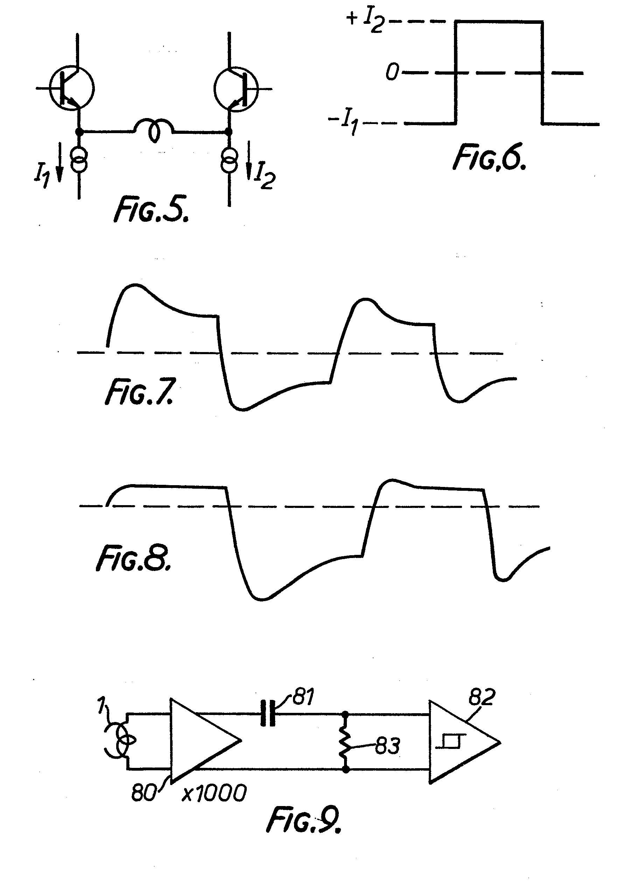

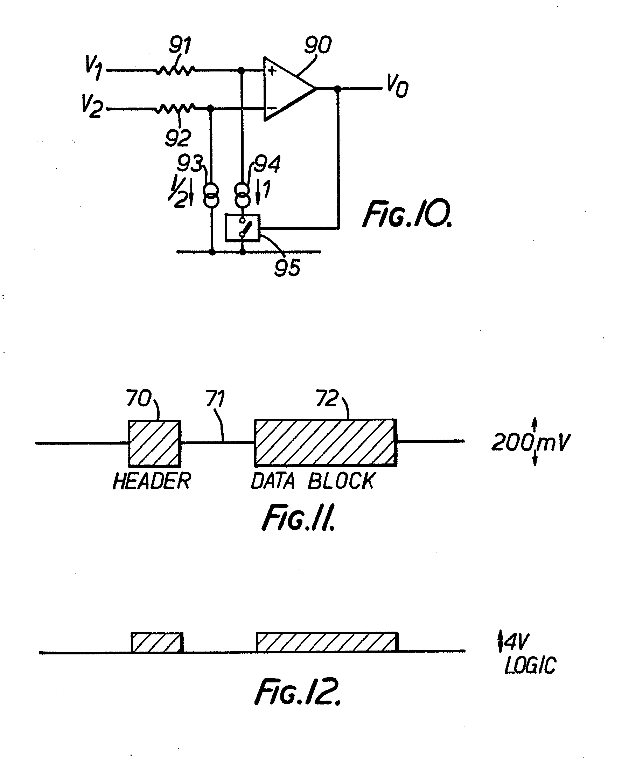

Referring now to Figure 1, there is shown part of an example of apparatus according to the invention with a cassette 2 of magnetic tape inserted into the aparatus by pushing it in the direction indicated by the arrow A through an aperture in the exterior casing B of the apparatus. The cassette 2 in the present instance contains magnetic tape 1 in a closed loop of about 20 feet in length on a single reel, not shown, which tape 1 is withdrawn from the centre of the reel and rewound on the outside, and from the reel, the tape 1 passes over a roller 3 which is preferably fixed against rotation so as to avoid any eccentricity of the roller influencing the movement of the tape. After passing over a transducing head 10 near the end wall of the cassette 2, the tape 1 passes round a free roller 4, which in use acts as a pinch roller, and then returns to the reel in the cassette. A pressure pad 5 is provided urged upwardly by a leaf spring 6 to press the tape 1 against the magnetic transducing head 10. The head 10 is mounted on a surface 11 of the body of the apparatus in a manner to be described later and the cassette 2 slides over this surface when it is inserted into the apparatus. In practice, a second surface is provided above the cassette as part of the outer casing of the apparatus, but this is not shown in the drawings.

A wall 7 is provided upstanding from the surface 11 which guides the cassette 2 as it is inserted into the apparatus and the upper left-hand corner 9 of the cassette is eased round the drive roller 12 by a ramp 8 on the wall 7. Spring 14 which urges the cassette 2 to the left causes the corner 9 to be pressed against the surface of the roller 12 tending to cause it to rotate in an anti-clockwise direction. It will be appreciated that the normal direction of rotation of the roller 12 is clockwise so as to draw the tape 1 past the head 10. Because of the inertia of the tape drive means, the rotation of the drive roller 12 in anti-clockwise direction when the cassette 2 is inserted into the apparatus may mean that the roller 12 is still rotating in an anti-clockwise direction when the tape 1 is held between the rollers 4 and 12, and consequently some of the tape 1 may be unwound from the outside of the reel in the casette 2. This unwinding of tape 1 could lead to creasing or other damage to the tape which could degrade its magnetic properties to an undesirable extent, bearing in mind the density of recording required on the tape. In order to stop this anti-clockwise rotation of the drive roller 12, the drive roller is provided with a part 16 with a ratchet surface which engages a pawl member 17 on a resilient arm 19. As the cassette 2 is pushed in, a limb 21 attached to the arm 19 engages the corner 9 of the cassette 2 which bends the pawl member 17 away from the ratchet part 16 when the cassette is pushed fully home.

When the cassette is fully home, its end wall meets fixed pegs 22, and its left-hand wall is urged against an abutment 15 on the wall 7. In this position, the pressure of the spring 14 pushes the cassette leftwards so that the tape 1 is held firmly between the drive roller 12 and the pinch roller 4. The tape gripping pressure also serves to hold the cassette in the apparatus because it has a significant component in the upward direction as shown in Figure 1. The pressure of the drive roller 12 also tends to rotate the cassette 2 about the right-hand one of the pegs 22 so as to hold the cassette against the abutment 15. Thus the position of the cassette 2 is detemined by the drive roller 12, the right-hand peg 22 and the abutment 15. From a mechanical point of view, the left-hand peg 22 is redundant but serves to provide an alternative end stop for the cassette 2 should the right-hand peg 22 become damaged, for example by too great an insertion pressure.

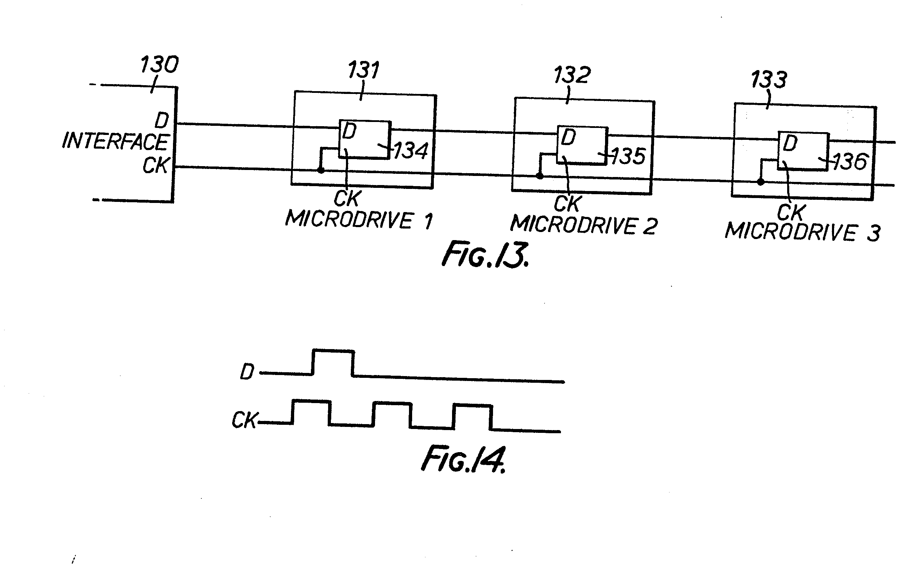

It is now necessary to consider the location of the cassette 2 in a direction normal to the plane of the paper as shown in Figure 1 and reference will now be made to Figures 2 and 3 as well as Figure 1.

The upper end of the cassette 2 engages leaf springs 23 on the surface 11 which urge the cassette 2 away from the surface 11. The transducing head 10 has side plates 28 provided with one or more projections, two bearing the references 25 and 26 being shown in Figure 2. The projections 25 and 26 have tapered or rounded ends to guide the tape 1 and the body of the cassette 2 in the manner to be described. The upper surface of the lower part of the casing of the cassette 2 engages the lower edge 25B of the projection 25 and is held against it by the pressure of the spring 23. In this way the position of the cassette 2 in the direction normal to the paper as shown in Figure 1 is determined. The tape 1 is guided by the upper edge 25A of the projection 25 and the lower edge 26A of the projection 26, so that the position of the tape 1 over the head 10 is fixed by each of the end plates 28, and therefore the direction followed by the tape 1 over the head 10 is also determined. The positions of the plates 28 on the sides of the head 10 are accurately determined so that the tape 1 passes squarely over the recording gaps and the longitudinal direction of the tape 1 is held to be closely at right angles to the head in Figure 2 and these represent the manner in which the plates 28 are attached to the sides of the head 10. The alignment of the head 10 relative to the surface 11 is determined by the legs 30 on the plates 28 which are held against the surface 11 by the manner of attachment of the head 10 to the surface 11.  The attachment itself is provided by a flexible metallic strip 31 which extends both sides of the head 10 and this strip is secured at its ends to the surface 11 using, for example, screws or rivets or pegs of plastics material.  The resilience of the metallic strip 31 serves to press the legs 30 against the surface 11.

As shown in Figure 1, the upper part of the casing of the cassette 2 is cut away to provide clearance for the upper tape guides 26 on the transducing head 10.  It has been found that the upper tape guides 26 can be omitted from the plate 28 on one or both sides of the head 10 if the apparatus is such that the tape 1 is biassed towards the lower tape guides 25.

Referring to Figure 4, a recording/playback arrangement includes a recording/playback head 1 one terminal of which is connected to the emitter electrode of a first NPN transistor 3.  The emitter electrode of the transistor 2 is connected also to a controllable current source 4 and the collector electrode is provided with a load resistor 5 connected to a voltage supply.  The base electrode of the transistor 2 is connected to a terminal for a waveform B when recording.  The transistor 3 has its own emitter current source 6 corresponding to the current source 4 for the transistor 2 and a collector load resistor 7 corresponding to the collector load resistor 5 for the transistor 2. The base electrode of the transistor 3 is connected to a terminal for a waveform A when recording. The current sources 4 and 6 may be set at a first, relatively high level of a few tens of milliamperes, or at a second, relatively low level of several tens of microamperes.

In the operation of the recording/playback arrangement of Figure 4, recording of a data signal is effected by driving the base electrode of the transistors 2 and 3 by means of the upright and inverted forms, B and A respectively, of the data signal and setting the respective current sources 4 and 6 at relatively high levels, referred to above. The current source settings are, in practice, not the same, for reasons set out below. The transistor 2 is therefore switched off when the transistor 3 is switched on, and vice-versa, with the result that the current from the current source 6 flows through the recording head 1 in one direction when the transistor 2 is switched on and the transistor 3 is switched off, and the current from the current source 4 flows through the recording head 1 in the opposite direction when the transistor 3 is switched on and the transistor 2 is switched off. An effect of arranging for the reversal of the magnetising current through the recording head 1 is that the peak-to-peak value of the drive voltage applied to the recording head 1 may exceed the supply voltage level to the collector loads of the transistors 2 and 3. For example, with a supply voltage of 5 volts, the drive voltage applied to the recording head 1 may be of the order of 8 volts.

In the operation of the recording/playback arrangement of Figure 4 in the playback mode, the base electrodes of the transistors 2 and 3 are connected to fixed bias voltages to provide low noise level bias, the current source currents are set at the relatively low current levels referred to above, and the transistors 2 and 3 are operated as low-level common-base amplifiers with a differntial output signal available from collector to collector. The arrangement facilitates construction with the recording head 1 connected directly to the pins of an integrated circuit including the remaining components, an effect of which is to minimise the amount of external noise which will find its way into the output signal.

The recording/playback arrangement of Figure 4 may be employed to effect the erasure of data by means of the recording head 1 by the use of a steady magnetising signal to effect saturation of the recording tape material. A consequence of d.c. erasure is that the recording tape material displays a magnetic bias after erasure. The technique, referred to above and illustrated by Figures 5 and 6, of recording with the current required by the current source 4 different from the current required by the current source 6, may be used to remove the magnetic bias to provide a recording centred about zero magnetic bias as illustrated by Figure 7. The technique is optional.

Figure 11 represents a signal which may be the differential output signal of the recording/playback arrangement of Figure 4 in the playback mode. Referring to Figure 11, the differential output signal consists of a header signal 70 followed by a gap 71 which is itself followed by a data signal 72, the header signal 70 and the data signal 72 each being of about 200 millivolts amplitude.

Referring to Figure 9, a circuit arrangement for obtaining a signal such as that represented by Figure 11 and for processing the signal includes a recording head 1, a differential amplifier 80, a coupling capacitor 81, a comparator 82, and a resistor 83 connected between the input ports of the comparator 82.

Referring to Figure 10, the comparator 82 (Fig.9) includes a differential amplifier 90 having a resistor 91 in series with its non-inverting input port and a resistor 92 in series with its inverting input port. The inverting input port of the differential amplifier 90 is connected to the input of a current source 93 the output of which is connected to the negative power supply rail. The non-inverting input port of the differential amplifier 90 is connected to a current source 94 the output of which is connected to the negative power supply rail by way of a switch device 95. The switch device is either open or closed according to the output voltage of the differential amplifier 90 and has a control input connected to the output port of the differential amplifier 90 for the purpose.

In the operation of the comparator represented by Figure 10, a negative or small positive differential input voltge applied to the resistors 91 and 92 will result in zero output voltage, the switch 95 being closed to cause current to flow through the resistor 91 and thereby hold the non-inverting input port of the amplifier 90 at a potential below that of its inverting input port. When the differential input voltge attains a high·enough positive value, the output voltage of the differential amplifier 90 will become positive and will result in the switch device 95 becoming an open circuit. The voltage at the non-inverting input port of the differential amplifier 90 will then rise by a further amount dependent on the value of the resistor 91 and the setting for the current source 94. The voltage at the non-inverting input port of the differential amplifier 90 will fall again by the, further amount dictated by the value of the resistor 91 and the setting of the current source 94 when the differential input voltage falls to zero.

Referring to Figures 9 and 10, it will be observed that the resistor 83 maintains the input ports of the comparator 82 at the same d.c. potential, ensuring that the bias conditions do not influence the behaviour of the comparator 82. Also, the hysteresis of the comparator 82 is defined by the value of the input resistor 91 and the setting of the current source 94.

Figure 12 represents a signal produced by the comparator 82 from the signal represented by Figure 11. In Figure 12, the gap between the active portions of Figure 11 has been preserved while the active portions have been amplified to several volts compatible with digital logic circuitry. In particular, the gap between the active portions may be recognised by digital logic circuitry, which may include a central processing unit, as a synchronisation period, occurring immediately before a data block.

Referring to Figure 13, three recording/playback devices 131, 132, 133 are shown connected to a control device 130 which is itself controllable by a computer, say, to select one of the three recording/playback devices 131, 132, 13 for operation. The recording/playback device 131 includes a D flip-flop 134, the D-input terminal of which is connected to a first output terminal of the control device 130 and the clock-input terminal of which is connected to the clock-output terminal of the control device 130. The second recording/playback device 132 includes a D flip-flop 135, the D-input terminal of which is connected to the Q-output terminal of the flip-flop 134, and the CLOCK-input terminal of which is connected to the CLOCK-input terminal of the flip-flop 131. The third recording/playback device 133 includes a D flip-flop 136, the D-input terminal of which is connected to the Q-output terminal of the flip-flop 135, and the CLOCK-input terminal of which is connected to the CLOCK-input terminal of the flip-flop 135.

In the operation of the arrangement shown in Figure 13, the flip-flops 131, 132, 133 behave as a shift register, permitting a logic 1 condition originating at the control device 130 to be loaded into the first flip-flop 131 by the supply of a single clock pulse from the control device 130, the logic 1 condition to move to the second flip-flop 135 by the supply of a further clock pulse, and the logic 1 condition to move to the third flip-flop 136 by the supply of yet another clock pulse. The recording/ playback device 131 is switched on when the flip-flop 131 is set at logic 1, the recording/playback device 132 is switched on when the flip-flop 135 is set at logic 1, and so on. The control device 130 is therefore able to select any one of the recording/ playback devices 131, 132, 133 by suplying a logic 1 condition accompanied by an apropriate number of clock pulses, Figure 14 illustrating the output required from the control device 130 to select the recording/ playback device 133.

From the above description of Figure 13, it will be appreciated that the control device 130 may be used to select associated devices other than recording/ playback devices and that any number of associated devices may be accommodated by use of the technique described.

Although the invention has been described with reference to a specific example, it will be appreciated that the apparatus may take different forms without departing from the invention.

0132918

WHAT WE CLAIM IS

1.    Magnetic tape recording and reproducing apparatus using magnetic tape in a cassette and having a magnetic transducing head and tape driving means with a drive roller bearing against the tape and a pinch roller in the cassette, wherein both the head and the drive roller are fixed in position in the apparatus, spring means is provided urging the cassette towards the drive roller and the drive roller is so disposed in relation to the cassette that the pressure of the drive roller serves to urge the cassette towards the head and also to retain the cassette in the apparatus.

2.    Magnetic tape recording and reproducing apparatus, as claimed in claim 1, including a first stop member beside the head and a second stop member beside the drive roller, which members under the influence of the spring means determine the position of the cassette when pushed home in the apparatus.

3.    Magnetic tape recording and reproducing apparatus, as claimed in claim 1 or claim 2, including means arranged to cooperate with a cassette to prevent a part of the cassette from causing the drive roller to rotate as the cassette is pushed home in the apparatus.

4.    Magnetic tape recording and reproducing

23

0132918

apparatus, as claimed in any one of the preceding claims, including a magnetic transducing head provided with tape guide members which contact one or both edges of the tape both upstream and downstream of the head gap, thereby determining the alignment of the tape over the head itself.

5. Magnetic tape recording and reproducing apparatus, as claimed in claim 4, including guide members arranged to determine the position of the cassette casing relative to the head.

6. Magnetic tape recording and reproducing apparatus, as claimed in claim 4, wherein the tape guide members are arranged also to determine the position of the cassette casing relative to the head.

7. Magnetic tape recording and reproducing apparatus, as claimed in any one of the preceding claims, wherein the head is positioned in relation to a surface parallel to the longitudinal direction of the tape in a cassette, the head including positioning side plates with legs which rest on the surface and means for urging the head resiliently towards the surface.

8. A magnetic tape recording and reproducing apparatus having a transducing head including guide members for determining the alignment of tape over the head, guide members for determining the position of a cassette relative to the head, and means for positioning the head in relation to a surface, and means for urging the head resiliently towards the surface.

9.      Magnetic recording and reproducing apparatus, as claimed in any one of the preceding claims, wherein th magnetic transducing head is so positioned as to contact the tape in a cassette along one edge of the cassette, and the drive roller is so positioned as to contact the tape along a different edge of the cassette.

10.     Magnetic tape recording and reproducing apparatus, as claimed in claim 9, wherein the magnetic transducing head and the drive roller are so positioned as to contact the tape along adjacent dges of the cassette.

11.     Magnetic tape recording and reproducing apparatus, as claimed in claim 9 or claim 10, including cassette guide means arranged to permit insertion of a tape cassette towards the transducing head.

12.     Apparatus according to any preceding claim, wherein the magnetic transducing head is connected from the output of a first current source to the output of a second current source, the outputs of the current sources also being connected to a reference voltage supply through respective switchable devices, the switchable devices being caused to conduct alternately to provide the recording energisation of the head.

13.     Apparatus according to claim 12, wherein the switchable devices are transistors to the control electrodes of which a recording waveform is applied in push-pull.

14. Apparatus according to claim 13, wherein during playback the control electrode are connected to a fixed voltage level so that the transistors amplify voltages generated by the head.

15. Apparatus according to claim 14 wherein the amplified voltages are applied to a comparator with a switchable current source connected to one input, the current source being switched by the output of the comparator.

0132918

1/5

*Fig.1.*

*Fig.2.*

Fig. 3.

Fig. 4.

FIG.5.

FIG.6.

FIG.7.

FIG.8.

FIG.9.

*Fig.10.*

*Fig.11.*

*Fig.12.*

130

131

132

133

INTERFACE

D

CK

D

134

CK
MICRODRIVE 1

D

135

CK
MICRODRIVE 2

D

136

CK
MICRODRIVE 3

*FIG.13.*

D

CK

*FIG.14.*